# FASCICULE DE BREVET EUROPEEN

(11) **EP 1 380 287 B1**
(45) Date de publication et mention de la délivrance du brevet: **10.08.2011**
(21) Numéro de dépôt: 03291573.8
(22) Date de dépôt: 26.06.2003
(51) Int. Cl.: A61Q 5/08, A61K 8/00, A61K 8/22, A61K 8/25, A61K 8/81, A61K 8/90

(54) **Pâte anhydre pour la décoloration des fibres kératiniques humaines**
Wasserfreie Paste zum Entfärben von menschlichen Keratinfasern
Anhydrous paste for bleaching human keratinous fibres

(30) Priorité: 12.07.2002 FR 0208855
(43) Date de publication de la demande: 14.01.2004
(73) Titulaire: L'Oréal, 75008 Paris (FR)
(72) Inventeur: Legrand, Frédéric, 92400 Courbevoie (FR)
(74) Mandataire: Wattremez, Catherine

(56) Documents cités:
- EP-A- 0 808 895
- WO-A-99/62469
- DE-A1- 3 814 356
- GB-A- 1 083 007
- US-A- 5 437 860
- US-A- 5 888 484

## Description

La présente invention concerne une pâte anhydre pour la décoloration des fibres kératiniques humaines, et en particulier des cheveux, comprenant au moins un sel peroxygéné, au moins un agent alcalin, 15 à 35% en poids d'au moins un polydécène de formule C₁₀ₙ H_{[(20n)+2]} dans laquelle n varie de 3 à 9 et de préférence de 3 à 7, et 0,01 à 10% en poids d'au moins un gélifiant choisi parmi les silices pyrogénées à caractère hydrophile ou hydrophobe ou un copolymère di-, tri-, multi- ou radial bloc composé de segments de type styrène monomère et de segments de type monomère ou comonomère thermoplastique.
L'invention concerne également le procédé de décoloration des fibres kératiniques humaines la mettant en oeuvre.

La décoloration des fibres kératiniques humaines et plus particulièrement des cheveux se fait par oxydation du pigment "mélanine" aboutissant à la solubilisation et l'élimination partielle ou totale de ce pigment.
Pour décolorer les cheveux, on utilise des poudres décolorantes contenant un réactif peroxygéné tel que les persulfates, perborates et percarbonates, d'ammonium ou de métaux alcalins, que l'on associe au moment de l'emploi à une composition aqueuse de peroxyde d'hydrogène.
Les sels peroxygénés et le peroxyde d'hydrogène étant relativement stables en milieu acide, il est souvent nécessaire de les activer à pH basique pour obtenir une formation adéquate d'oxygène. Il est donc usuel d'ajouter aux poudres décolorantes, des composés alcalins tels que l'urée, les silicates et les phosphates de métaux alcalins ou alcalino-terreux, et en particulier les métasilicates de métaux alcalins, ou des agents précurseurs d'ammoniac comme les sels d'ammonium .

On sait que les poudres décolorantes ont tendance à former de la poussière durant leur manutention, leur transport et leur stockage.
Or, les produits qui les composent (persulfates, silicates alcalins) sont corrosifs, irritants pour les yeux, les voies respiratoires et les muqueuses. C'est pourquoi on a proposé de réduire le taux de poussière en utilisant divers moyens :
- en déposant sur la poudre, par un procédé de pulvérisation, divers composés substantiellement insolubles dans l'eau tels que par exemple des huiles ou des cires liquides, lesquels en enrobant les particules de la poudre, les agglomèrent en des particules de taille plus grande. Le brevet EP-560088 décrit un tel procédé,
- en utilisant des polymères, anhydres, liquides et solubles dans l'eau à température ambiante, tels que ceux choisis parmi les polyéthylèneglycols ou les polypropylèneglycols (brevet français N° 2 716 804), ou ceux linéaires séquencés bloc et/ou statistique du type polyoxyéthylène/polyoxypropylène (brevet EP-663 205),
- en utilisant, comme dans le brevet américain N°-5866107, des composés tels que la γ-butyrolactone, le diméthyl isosorbide, le diisopropyl isosorbide, ou des diesters d'alkyles en C₃-C₆.

Pour s'affranchir du problème de volatilité des poudres décolorantes, on développe maintenant des pâtes qui comprennent lesdits agents pulvérulents (sels peroxygénés, agents alcalins, épaississants) dans un support liquide inerte organique épaissi. De telles compositions sont notamment décrites dans les demandes de brevets DE-3814 356 A1, DE-197 23 538 C1 et le brevet américain N° 4 170 637.
Les pâtes décolorantes basées sur cette technologie et qui sont actuellement sur le marché, renferment de l'huile minérale à titre de liquide inerte. Or de telles compositions comprenant de l'huile minérale communiquent aux cheveux un toucher gras après traitement.
Elles présentent en outre une stabilité physico-chimique non satisfaisante et ne permettent pas d'obtenir une décoloration suffisamment homogène et puissante. Par ailleurs, elles ne présentent pas un bel aspect.
Pour épaissir l'huile minérale et améliorer la stabilité, on a utilisé des cires, dont notamment des produits dont le point de fusion est supérieur à 40°C ou des esters d'acides gras hydrophobes à chaîne longue ou des produits de substitution de la cire d'abeille.
Mais dans ces conditions, pour être dispersée, voire solubilisée dans le liquide organique, la cire doit être fondue, ce qui implique de la chauffer au cours du procédé de fabrication.
Or la Demanderesse a constaté que de telles compositions à base de cires sont sensibles à la température et aux chocs thermiques, tant au cours de leur fabrication que de leur stockage. Les pâtes perdent alors leurs qualités d'usage lorsqu'elles sont exposées à de grandes variations de température ou à des températures extrêmes (inférieures ou égales à 4°C ou supérieures ou égales à 45°C).
Voici maintenant, qu'après d'importantes recherches menées sur la question, la Demanderesse a découvert qu'en utilisant un polydécène de formule C₁₀ₙ H_{[(20n)+2]} dans laquelle n varie de 3 à 9 et de préférence de 3 à 7 et un agent gélifiant choisi parmi les silices pyrogénées à caractère hydrophile ou hydrophobe ou les polymères blocs comprenant au moins un motif alkylène ou oxyde d'alkylène, elle obtenait des pâtes décolorantes anhydres stables sur le plan physico-chimique, qui en outre ne laissent pas un toucher gras après décoloration.

Les pâtes décolorantes présentent alors l'avantage d'être préparées sans être chauffées; elles sont préparées à froid ou à température ambiante et sont moins sensibles à la température et aux chocs thermiques en cours de fabrication ou au stockage.
Par ailleurs, elles permettent d'obtenir des décolorations puissantes et homogènes, en ne laissant les cheveux ni gras ni rêches.

Cette découverte est à la base de la présente invention.

La présente invention a ainsi pour objet une pâte anhydre pour la décoloration des fibres kératiniques humaines, et plus particulièrement des cheveux, comprenant au moins un sel peroxygéné, au moins un agent alcalin, caractérisée par le fait qu'elle comprend 15 à 35% en poids d'au moins un polydécène de formule C₁₀ₙ H_{[(20n)+2]} dans laquelle n varie de 3 à 9 et de préférence de 3 à 7, et 0,01 à 10% en poids d'au moins un gélifiant choisi parmi les silices pyrogénées à caractère hydrophile ou hydrophobe ou un copolymère di-, tri-, multi- ou radial bloc composé de segments de type styrène monomère et de segments de type monomère ou comonomère thermoplastique.

Elle a également pour objet l'utilisation de ladite pâte pour la préparation d'une composition de décoloration prête à l'emploi.
Par "composition prête à l'emploi" on entend, au sens de l'invention, la composition destinée à être appliquée telle quelle sur les fibres kératiniques, c'est à dire qu'elle résulte du mélange extemporané de la pâte et de la composition aqueuse de peroxyde d'hydrogène.
Par "anhydre", on entend, au sens de l'invention, une pâte dont la teneur en eau est inférieure à 1%, et de préférence inférieure à 0,5% en poids par rapport au poids total de la pâte.

L'invention vise également un procédé de décoloration des fibres kératiniques humaines, et plus particulièrement des cheveux utilisant la composition de décoloration prête à l'emploi telle que décrite selon l'invention.

Un autre objet de l'invention est un dispositif à plusieurs compartiments pour ladite composition de décoloration prête à l'emploi comportant au moins deux compartiments dont l'un contient une pâte anhydre et l'autre une composition aqueuse de peroxyde d'hydrogène.

Mais d'autres caractéristiques, aspects, objets et avantages de l'invention apparaîtront encore plus clairement à la lecture de la description et des exemples qui suivent.

### Polydécènes de formule C₁₀ₙH_{[(20n)+2]} avec n variant de 3 à 9.

Ces composés répondent à l'appellation "polydécène" du Dictionnaire CTFA 7ème édition 1997 de la Cosmetic, Toiletry and Fragrance Association, USA, ainsi qu'à la même appellation I.N.C.I. aux USA et en Europe.
Ce sont des produits d'hydrogénation des poly-1-décènes.

Parmi ces composés, on choisit plus particulièrement selon l'invention ceux pour lesquels dans la formule, n varie de 3 à 7.

On peut citer, à titre d'exemple, et de préférence, le produit vendu sous la dénomination SILKFLO® 366 NF POLYDECENE par la société AMOCO CHEMICAL, ceux vendus sous la dénomination NEXBASE® 2002 FG, 2004 FG, 2006 FG et 2008 FG par la société FORTUM.

Dans la pâte décolorante selon l'invention, le ou les polydécènes sont présents de préférence dans une proportion pondérale allant d'environ 15 à 30% et plus particulièrement d'environ 15 à 25% par rapport au poids total de la pâte.

### Agent gélifiant

Parmi les silices pyrogénées à caractère hydrophile utilisables selon la présente invention, on peut citer notamment celles vendues par la société DEGUSSA HÜLS sous les dénominations commerciales AEROSIL® 90, 130, 150, 200, 300 et 380.
Parmi les silices pyrogénées à caractère hydrophobe utilisables selon la présente invention, on peut citer notamment celles vendues par la société DEGUSSA HÜLS sous les dénominations commerciales AEROSIL® R202, R805, R812, R972 et R974.
Les copolymères di-, tri-, multi- ou radial blocs composés de segments de type styrène monomère et de segments de type monomère ou comonomère thermoplastique sont décrits dans le brevet US-5 221 534.
Parmi ces copolymères blocs, on préfère utiliser notamment ceux pour lesquels le monomère ou comonomère thermoplastique désigne éthylène/alkylène en C₃-C₄, et plus particulièrement encore un copolymère hydrogéné à blocs styrène et à blocs éthylène/alkylène en C₃-C₄.
On utilise plus avantageusement encore selon l'invention, un mélange de copolymère hydrogéné à blocs butylène/éthylène et à blocs styrène et de copolymère hydrogéné à blocs éthylène/propylène et à blocs styrène, dans de l'huile minérale, et notamment un mélange de 1 à 20% en poids de copolymère hydrogéné à blocs butylène/éthylène et à blocs styrène et de copolymère hydrogéné à blocs éthylène/propylène et à blocs styrène dans 80 à 99% en poids d'huile minérale.
De tels mélanges sont par exemple vendus par la société PENRECO sous les dénominations commerciales VERSAGEL® M200 et GEAHLENE® 200 et VERSAGEL® M750 et GEAHLENE® 750, ou par la société AIGLON sous les dénominations commerciales TRANSGEL® ou SYNGEL® (90% d'huile de paraffine, 5% de copolymère hydrogéné butylène/éthylène/styrène, 5% de copolymère hydrogéné éthylène / propylène / styrène).
On peut également utiliser les polymères tri-blocs styrène/éthylene-butylène/styrène (nom I.NC.I. "Hydrogenated styrene/butadiene copolymer") vendus par la société SHELL CHIMIE sous les dénominations commerciales KRATON® G-1650, G-1652 et G-1657.

Dans les pâtes selon l'invention, le ou les agents gélifiants sont de préférence présents à une concentration allant d'environ 0,01 à 10%, de préférence d'environ 0,01 à 5% et plus particulièrement d'environ 0,1 à 2,5% en poids par rapport au poids total de la pâte.

### Sels peroxygénés.

Comme indiqué ci-dessus, ils sont choisis parmi les persulfates, les perborates et les percarbonates d'ammonium ou de métaux alcalins ainsi que le peroxyde de magnésium et les mélanges de ces composés.
On utilise de préférence les persulfates, et parmi ceux-ci principalement les persulfates de sodium et de potassium
Dans les pâtes décolorantes selon l'invention, le ou les sels peroxygénés peuvent être présents à une concentration allant d'environ 10 à 70% et de préférence d'environ 20 à 60% par rapport au poids total de la pâte.

### Agents alcalins

Ces agents alcalins déjà décrits ci-dessus (urée, silicates et phosphates de métaux alcalins ou alcalino-terreux, et en particulier métasilicates de métaux alcalins, ou agents précurseurs d'ammoniac comme les sels d'ammonium), peuvent être présents dans les pâtes décolorantes à une concentration allant d'environ 0,01 à 40% et de préférence d'environ 0,1 à 30% en poids par rapport au poids total de la pâte.
Selon une alternative, ils peuvent être présents dans une composition aqueuse à mélanger au moment de l'emploi à la composition aqueuse de peroxyde d'hydrogène.

Un autre objet selon la présente invention est une pâte anhydre pour la décoloration des fibres kératiniques humaines, et plus particulièrement des cheveux, comprenant 10 à 70% en poids d'au moins un persulfate de sodium ou de potassium, 0,01 à 40% en poids d'au moins un agent alcalin, 15 à 35% en poids d'au moins un polydécène de formule C₁₀ₙ H_{[(20n)+2]} dans laquelle n varie de 3 à 9 et de préférence de 3 à 7, et 0,01 à 10% en poids d'au moins un gélifiant choisi parmi les silices pyrogénées à caractère hydrophile ou hydrophobe et les copolymères blocs hydrogénés à blocs styrène et à blocs éthylène/alkylène en C₃-C₄.

De préférence, la pâte selon l'invention comprend en outre au moins un polymère amphiphile comportant au moins un chaîne grasse de type non ionique ou anionique.

Les polymères amphiphiles convenant à la mise en oeuvre de la présente invention sont notamment les suivants :

### Polymères amphiphiles non ioniques comportant au moins une chaîne grasse

Ils sont choisis de préférence parmi :
- **(1)** les celluloses modifiées par des groupements comportant au moins une chaîne grasse ; on peut citer à titre d'exemple :

- les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS (alkyles en C₁₆) vendu par la société AQUALON, ou le produit BERMOCOLL EHM 100 vendu par la société BEROL NOBEL,
- celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.
- **(2)** les hydroxypropylguars modifiés par des groupements comportant au moins une chaîne grasse en C₈ à C₂₂, tel que le produit JAGUAR XC-95/3 (chaîne alkyle en C₁₄) vendu par la société RHODIA, le produit ESAFLOR HM 22 (chaîne alkyle en C₂₂) vendu par la société LAMBERTI, les produits RE210-18 (chaîne alkyle en C₁₄) et RE205-1 (chaîne alkyle en C₂₀) vendus par la société RHONE POULENC.
- **(3)** les copolymères de vinyl pyrrolidone et de monomères hydrophobes à chaîne grasse dont on peut citer à titre d'exemple :
- les produits ANTARON V216 ou GANEX V216 (copolymère vinylpyrrolidone / hexadécène) vendu par la société I.S.P.
- les produits ANTARON V220 ou GANEX V220 (copolymère vinylpyrrolidone / eicosène) vendu par la société I.S.P.
- **(4)** les copolymères de méthacrylates ou d'acrylates d'alkyles en C₁-C₆ et de monomères amphiphiles comportant au moins une chaîne grasse.
- **(5)** les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
- **(6)** les polymères à squelette aminoplaste éther possédant au moins une chaîne grasse, tels que les composés PURE THIX proposés par la société SUD-CHEMIE.
- **(7)**les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.
Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.
Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.
Par extension figurent aussi parmi les polyéthers polyuréthanes non-ioniques à chaîne grasse, ceux dont les séquences hydrophiles sont liées aux séquences lipophiles par d'autres liaisons chimiques.
A titre d'exemples de polyéthers polyuréthanes non-ioniques à chaîne grasse utilisables dans l'invention, on peut citer le Ser-ad FX 1100 de la société SERVO DELDEN qui est un copolymère dénommé sous la désignation I.N.C.I. européenne et américaine "Steareth-100/PEG-136/H.M.D.I. Copolymer".
On peut aussi utiliser le Rhéolate 205 à fonction urée vendu par la société RHEOX ou encore les Rhéolates 208 , 204 ou 212, ainsi que l'Acrysol RM 184.
On peut également citer le produit ELFACOS T210 à chaîne alkyle en C₁₂₋₁₄ et le produit ELFACOS T212 à chaîne alkyle en C₁₈ de chez AKZO.
Les polyéthers polyuréthanes utilisables selon l'invention sont en particulier ceux décrits dans l'article de G. Fonnum, J. Bakke et Fk. Hansen - Colloid Polym. Sci 271, 380.389 (1993).
On préfère en particulier les polyéthers polyuréthanes comportant au moins une chaîne grasse en C₁₀ à C₂₀, et les hydroxypropylguars modifiés par des groupements comportant au moins une chaîne grasse en C₈ à C₂₂.

### Polymères amphiphiles anioniques comportant au moins une chaîne grasse

Les polymères amphiphiles anioniques comportant au moins une chaîne grasse utilisables selon la présente invention sont des polymères réticulés ou non réticulés, comprenant :
- des motifs hydrophiles dérivés d'un ou de plusieurs monomères à insaturation éthylénique portant une fonction acide carboxylique libre, ou une fonction sulfonique libre ou partiellement ou totalement neutralisée, et
- des motifs hydrophobes dérivés d'un ou de plusieurs monomères à insaturation éthylénique portant une chaîne latérale hydrophobe, et éventuellement
- des motifs de réticulation dérivés d'un ou plusieurs monomères polyinsaturés.

- **(I)** Le ou les monomères à insaturation éthylénique portant une fonction acide carboxylique sont choisis parmi l'acide éthacrylique, l'acide méthacrylique et l'acide acrylique, de préférence parmi l'acide méthacrylique, l'acide acrylique et des mélanges de ceux-ci.

Le ou les monomères à insaturation éthylénique portant une chaîne latérale hydrophobe peuvent être (i) des esters d'acides carboxyliques insaturés et d'alcools gras, ou (ii) des éthers d'allyle et d'alcools gras.
(i) Les esters d'acides carboxyliques insaturés et d'alcools gras sont choisis par exemple parmi les éthacrylates, méthacrylates et/ou acrylates d'alkyles en C₁₀₋₃₀, de préférence en C₁₂₋₂₂. Ils englobent par exemple l'acrylate de lauryle, l'acrylate de stéaryle, l'acrylate de décyle, l'acrylate d'isodécyle, l'acrylate de dodécyle, et les méthacrylates correspondants, à savoir le méthacrylate de lauryle, le méthacrylate de stéaryle, le méthacrylate de décyle, le méthacrylate d'isodécyle, et le méthacrylate de dodécyle.
(ii) Les éthers allyliques d'alcools gras formant les motifs hydrophobes des polymères amphiphiles anioniques de la présente invention correspondent à la formule (I) suivante :

   CH₂= CR'CH₂OBₙR (I)
dans laquelle R' désigne H ou CH₃, B désigne le radical éthylèneoxy, n est nul ou désigne un entier allant de 1 à 100, R désigne un radical hydrocarboné choisi parmi les radicaux alkyle, arylalkyle, aryle, alkylaryle, cycloalkyle, comportant de 8 à 30 atomes de carbone, de préférence 10 à 24, et plus particulièrement encore de 12 à 18 atomes de carbone. Un motif de formule (I) plus particulièrement préféré est un motif dans lequel R' désigne H, n est égal à 10, et R désigne un radical stéaryle(C₁₈).
Ledit monomère réticulant est un composé comportant au moins deux doubles liaisons polymérisables non conjuguées l'une avec l'autre. On peut citer à tire d'exemple le phtalate de diallyle, le (méth)acrylate d'allyle, le divinylbenzène, le diméthacrylate de (poly)éthylèneglycol, le méthylène-bis-acrylamide, le polyallylsucrose ou le polyallylpentaérythritol.
Des polymères amphiphiles anioniques du type décrit ci-dessus sont décrits et préparés, par exemple dans les brevets US-3 915 921 et US-4 509 949 (copolymères d'acide (méth)acrylique et de (méth)acrylates d'alkyles en C₁₀₋₃₀, ou dans le brevet EP-0216479 B2 (copolymères d'acide (méth)acrylique et d'éthers allyliques d'alcools gras).

On peut citer à titre d'exemples de polymères préférés :
- les polymères réticulés d'acide acrylique et de méthacrylate d'alkyle en C₁₀₋₃₀, tels que le CARBOPOL ETD-2020 commercialisé par la société GOODRICH;
- les polymères réticulés d'acide acrylique et d'acrylate d'alkyle en C₁₀₋₃₀, tels que les polymères commercialisés sous les dénominations CARBOPOL 1382, PEMULEN TR1, PEMULEN TR2 par la société GOODRICH;
- le terpolymère acide méthacrylique/acrylate d'éthyle/méthacrylate de stéaryle oxyéthyléné (55/35/10);
- le terpolymère acide (méth)acrylique/acrylate d'éthyle/méthacrylate de béhényle oxyéthyléné 25 OE, et
- le terpolymère réticulé acide méthacrylique/acrylate d'éthyle/stéareth-10 allyl éther.
- **(II)** les polymères amphiphiles comportant comme motifs hydrophiles au moins un monomère à insaturation éthylénique à groupement sulfonique, sous forme libre ou partiellement ou totalement neutralisée et au moins une partie hydrophobe sont par exemple décrits dans les demandes de brevets français de la demanderesse N°-0016954 et 0100328 dont le contenu fait partie intégrante de la présente invention.

On peut notamment citer plus particulièrement parmi eux :
le copolymère acide acrylamido-2-méthyl-2-propane-sulfonique(AMPS) / n-dodécylacrylamide neutralisé par la soude, le copolymère réticulé par du méthylène-bis-acrylamide constitué de 75% en poids de motifs AMPS neutralisés par NH₃ et de 25% en poids de motifs acrylate de GENAPOL T-250, le copolymère réticulé par du méthacrylate d'allyle constitué de 90% en poids de motifs AMPS neutralisés par NH₃ et de 10% en poids de motifs méthacrylate de GENAPOL T-250, ou le copolymère réticulé par du méthacrylate d'allyle constitué de 80% en poids de motifs AMPS neutralisés par NH₃ et de 20% en poids de motifs méthacrylate de GENAPOL T-250.

Dans les pâtes décolorantes selon l'invention, le ou les polymères amphiphiles non ioniques et/ou anioniques comportant au moins une chaîne grasse peuvent être présents à une concentration allant d'environ 0,01 à 30% et de préférence d'environ 0,01 à 15% en poids par rapport au poids total de la pâte décolorante.

### Autres adjuvants

La pâte décolorante selon l'invention peut en outre comprendre des polymères épaississants hydrosolubles, des charges telles que des argiles ou de la silice amorphe, des liants tels que la vinylpyrrolidone, des lubrifiants comme les stéarates de polyol ou les stéarates de métaux alcalins ou alcalino-terreux, ainsi que des agents de contrôle du dégagement d'oxygène tels que le carbonate ou l'oxyde de magnésium, des agents colorants ou des agents matifiants comme les oxydes de titane ou encore des agents tensioactifs anioniques, non ioniques cationiques ou amphotères.

### Polymères épaississants hydrosolubles

Selon l'invention, ils englobent tous les polymères hydrosolubles synthétiques ou d'origine naturelle utilisés classiquement dans le domaine cosmétique et différents des polymères amphiphiles non ioniques et/ou anioniques comportant au moins une chaîne grasse de la présente invention décrits ci-dessus.
Comme exemples de polymères synthétiques, on peut citer, la polyvinylpyrrolidone, l'acide polyacrylique, le polyacrylamide, l'acide poly-2-acrylamidopropanesulfonique non réticulé comme par exemple le produit vendu sous la dénomination SIMUGEL EG par la société SEPPIC, l'acide poly-2-acrylamido-2-méthylpropane sulfonique réticulé, l'acide poly-2-acrylamido-2-méthylpropane sulfonique réticulé et partiellement neutralisé par l'ammoniaque vendu sous la marque HOSTACERIN AMPS par la société CLARIANT, des mélanges à effet épaississant synergique de l'acide poly-2-acrylamido-2-méthylpropane sulfonique non réticulé avec des éthers d'hydroxyalkylcellulose ou avec des poly(oxyde d'éthylène) tel qu'ils sont décrits dans le brevet US-4540510, ou des mélanges à effet épaississant synergique d'un acide poly(méth)acrylamido-alkyl(C₁-C₄)-sulfonique de préférence réticulé avec un copolymère réticulé de l'anhydride maléique et d'un alkyl(C₁-C₅)vinyléther tels que le mélange HOSTACERIN AMPS / STABILEZE QM (de la société ISF) et tels qu'ils sont décrits dans la demande de brevet français de la demanderesse N°-0014416.

Les polymères épaississants d'origine naturelle utilisables selon la présente invention, sont de préférence des polymères comportant au moins un motif sucre, à savoir : les gommes de guar non-ioniques ; les gommes de biopolysaccharides d'origine microbienne telles que les gommes de Scléroglucane ou de Xanthane ; les gommes issues d'exudats végétaux telles que les gommes Arabique, Ghatti, Karaya, Tragacanthe, Carrageenane, Agar et Caroube ; les pectines ; les alginates ; les amidons; les hydroxyalkyl(C₁-C₆)celluloses et carboxyalkyl(C₁-C₆)celluloses.
Par "motif sucre", on désigne au sens de la présente invention, une portion monosaccharidique (i.e. monosaccharide ou oside ou sucre simple) ou une portion oligosaccharidique (chaînes courtes formées de l'enchaînement d'unités monosaccharidiques, éventuellement différentes) ou une portion polysaccharidique [longues chaînes constituées d'unités monosaccharidiques, éventuellement différentes, i.e. polyholosides ou polyosides (homopolyosides ou hétéropolyosides)]. Les unités saccharidiques peuvent être en outre substituées par des radicaux alkyle, ou hydroxyalkyle, ou alcoxy, ou acyloxy, ou carboxyle, les radicaux alkyle comportant de 1 à 4 atomes de carbone.

Les gommes de guar non ioniques peuvent être modifiées ou non modifiées.
Les gommes de guar non modifiées sont par exemple les produits vendus sous la dénomination GUARGEL D/15 par la société GOODRICH, VIDOGUM GH 175 par la société UNIPECTINE, et sous les dénominations MEYPRO-GUAR 50 et JAGUAR C par la société MEYHALL.
Les gommes de guar non-ioniques modifiées sont notamment modifiées par des groupements hydroxyalkyle en C₁-C₆.
Parmi les groupements hydroxyalkyle, on peut mentionner à titre d'exemple, les groupements hydroxyméthyle, hydroxyéthyle, hydroxypropyle et hydroxybutyle.
Ces gommes de guar sont bien connues de l'état de la technique et peuvent, par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.
Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2.
De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société MEYHALL ou sous la dénomination GALACTASOL 4H4FD2 par la société AQUALON.

Les gommes de biopolysaccharides d'origine microbienne telles que les gommes de scléroglucane ou de xanthane, les gommes issues d'exudats végétaux telles que les gommes Aabique, gomme Ghatti, gomme Karaya, Tragacanthe, Carrageenane, Agar et

Caroube, les hydroxyalkylcelluloses et les carboxyméthylcelluloses, les pectines, les alginates et les amidons sont bien connus de l'homme de l'art et décrits notamment dans l'ouvrage de Robert L. DAVIDSON intitulé "Handbook of Water soluble gums and resins" édité chez Mc Graw Hill Book Company (1980).
Parmi ces gommes, les scléroglucanes sont représentés par les produits vendus sous la dénomination ACTIGUM CS par la société SANOFI BIO INDUSTRIES et en particulier ACTIGUM CS 11 et sous la dénomination AMIGEL par la société ALBAN MULLER INTERNATIONAL. D'autres scléroglucanes tels que celui traité au glyoxal dans la demande de brevet français N°2633940 peuvent également être utilisés.
Les Xanthanes sont représentés par les produits vendus sous les dénominations KELTROL, KELTROL T, KELTROL TF, KELTROL BT, KELTROL RD, KELTROL CG par la société NUTRASWEET KELCO, ou sous les dénominations RHODICARE S, RHODICARE H par la société RHODIA CHIMIE.

Parmi les dérivés d'amidon, on peut citer par exemple le produit vendu sous la dénomination PRIMOGEL par la société AVEBE.

Les hydroxyalkyl(C₁-C₆)celluloses sont plus particulièrement des hydroxyéthylcelluloses telles que celles vendues sous les dénominations CELLOSIZE QP3L, CELLOSIZE QP4400H, CELLOSIZE QP30000H, CELLOSIZE HEC30000A, CELLOSIZE POLYMER PCG10, par la société AMERCHOL, ou NATROSOL 250HHR, NATROSOL 250MR, NATROSOL 250M, NATROSOL 250HHXR, NATROSOL 250HHX, NATROSOL 250HR, NATROSOL HX, par la société HERCULES, ou encore TYLOSE H1000 par la société HOECHST.
Les hydroxyalkyl(C₁-C₆)celluloses sont également plus particulièrement des hydroxypropylcelluloses comme les produits vendus sous les dénominations KLUCEL EF, KLUCEL H, KLUCEL LHF, KLUCEL MF, KLUCEL G, par la société AQUALON.
Parmi les carboxyalkyl(C₁-C₆)celluloses, on utilise de préférence la carboxyméthylcellulose dont on peut citer les produits vendus sous les dénominations BLANOSE 7M8/SF, BLANOSE RAFFINEE 7M, BLANOSE 7LF, BLANOSE 7MF, BLANOSE 9M31F, BLANOSE 12M31XP, BLANOSE 12M31P, BLANOSE 9M31XF, BLANOSE 7H, BLANOSE 7M31, BLANOSE 7H3SXF, par la société AQUALON, ou encore AQUASORB A500 et AMBERGUM 1221, par le société HERCULES, ou encore CELLOGEN HP810A et CELLOGEN HP6HS9, par la société MONTELLO, ou encore PRIMELLOSE par la société AVEBE.

Lorsqu'ils ils sont présents dans les pâtes décolorantes de la présente invention, les polymères épaississants hydrosolubles sont présents dans une proportion pondérale allant d'environ 0,01 à 30% et de préférence d'environ 0,01 à 15% en poids par rapport au poids total de la pâte.

### Tensioactifs

Les tensioactifs convenant à la mise en oeuvre de la présente invention sont notamment les suivants :

### Tensioactifs anioniques

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle
ou benzyle. Parmi les tensioactifs anioniques encore utilisables, on peut également citer les sels d'acides gras tels que les sels des acides oléique, ricinoléique, palmitique, des acides d'huile de coprah ou d'huile de coprah hydrogénée et de préférence notamment les sels de sodium, calcium ou magnésium de l'acide stéarique; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### Tensioactifs non ioniques :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀- C₁₄) amines ou les oxydes de N-acylaminopropylmorpholine.

### Tensioactifs amphotères ou zwittérioniques :

Les agents tensioactifs amphotères ou zwitterioniques peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.
Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives: R₂ -CONHCH₂CH₂ -N(R₃)(R₄)(CH₂COO⁻)
dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle; et R₂'-CONHCH₂CH₂-N(B)(C)
dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂- CHOH - SO₃H
R₂' désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHODIA CHIMIE.

### Tensioactifs cationiques :

Parmi les tensioactifs cationiques on peut citer en particulier (liste non limitative) : les sels d'amines grasses primaires, secondaires ou tertiaires, éventuellement polyoxyalkylénées; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium; les dérivés d'imidazoline ; ou les oxydes d'amines à caractère cationique.

Lorsqu'ils sont présents, les agents tensioactifs représentent 0,01 à 40% et de préférence de 0,1 à 30% en poids par rapport au poids total de la composition.

La pâte décolorante selon l'invention peut aussi comprendre des polymères conditionneurs cationiques ou amphotères anhydres bien connus de l'homme de l'art et décrits dans les brevets français N°-2788974 et 2788976 et tels que décrits ci-après.

### Polymères cationiques

Au sens de la présente invention, l'expression "polymère cationique" désigne tout polymère contenant des groupements cationiques et/ou des groupements ionisables en groupements cationiques.
Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863. Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.
Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.
Ce sont des produits connus. Ils sont notamment décrits dans les brevets français n° 2 505 348 ou 2 542 997. Parmi lesdits polymères, on peut citer :
**(1)** Les homopolymères ou copolymères dérivés d'esters ou d'amides acryliques
   ou méthacryliques et comportant au moins un des motifs de formules (I), (II), (III) ou (IV) suivantes: 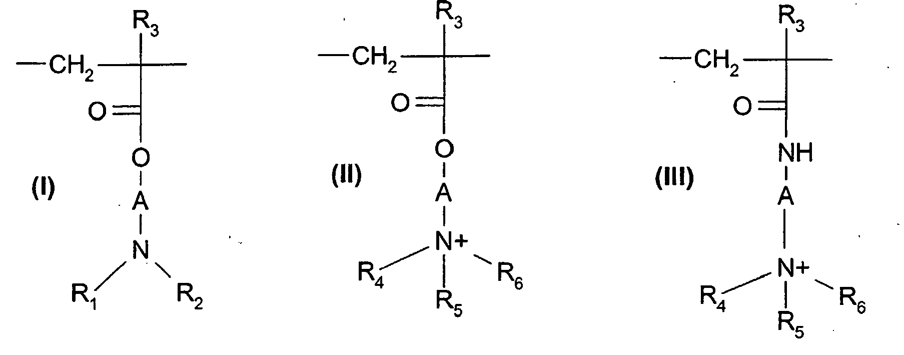 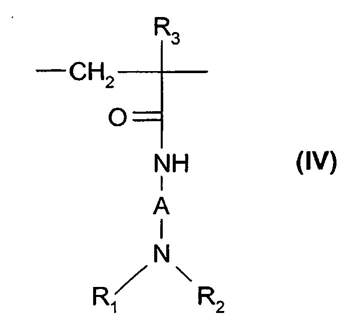 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.
   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle;
   - les copolymères d'acrylamide et de chlorure de méthacryloyloxyéthyltriméthylammonium décrits par exemple dans la demande de brevet EP-A-080976;
   - le copolymère d'acrylamide et de méthosulfate de méthacryloyloxyéthyltriméthylammonium;
   - les copolymères vinylpyrrolidone / acrylate ou méthacrylate de dialkylaminoalkyle quaternisés ou non. Ces polymères sont décrits en détail dans les brevets français 2.077.143 et 2.393.573;
   - les terpolymères méthacrylate de diméthyl amino éthyle/ vinylcaprolactame / vinylpyrrolidone;
   - les copolymère vinylpyrrolidone / méthacrylamidopropyl diméthylamine;
   - et les copolymères vinylpyrrolidone / méthacrylamide de diméthylaminopropyle quaternisés.
**(2)** Les dérivés d'éthers de cellulose comportant des groupements ammonium quaternaire décrits dans le brevet français 1 492 597. Ces polymères sont également définis dans le dictionnaire CTFA comme des ammonium quaternaires d'hydroxyéthylcellulose ayant réagi avec un époxyde substitué par un groupement triméthylammonium .
**(3)** Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropylcelluloses greffées notamment avec un sel de méthacryloyléthyl triméthylammonium, de méthacrylmidopropyl triméthylammonium, de diméthyl-diallylammonium.
**(4)** Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
**(5)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
**(6)** Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
**(7)** Les dérivés de polyaminoamides résultant de la condensation de polyalcoylènes polyamines avec des acides polycarboxyliques suivie d'une alcoylation par des agents bifonctionnels. On peut citer par exemple les polymères acide adipiquediacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine.
**(8)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
**(9)** Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 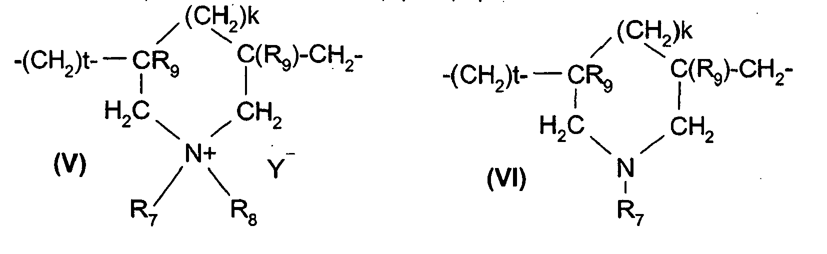 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
**(10)** Le polymère de diammonium quaternaire contenant des motifs récurrents répondant à la formule : 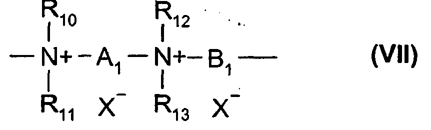 formule (VII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ; A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un
   ou plusieurs atomes d'oxygène, de soufre ou des groupements sulfoxyde, sulfone, disulfure, amino, alkylamino, hydroxyle, ammonium quaternaire, uréido, amide ou ester, et X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement-(CH₂)n-CO-D-OC-(CH₂)n- dans lequel n est compris entre 1 et 100 et de préférence entre 1 et 50, et D désigne :
   a) un reste de glycol de formule : -O-Z-O-, où Z désigne un radical hydrocarboné linéaire ou ramifié ou un groupement répondant à l'une des formules suivantes :

      -(CH₂-CH₂-O)x-CH₂-CH₂-

      -[CH₂-CH(CH₃)-O]y-CH₂-CH(CH₃)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH₂-CH₂-S-S-CH₂-CH₂-;
   d) un groupement uréylène de formule : -NH-CO-NH- .
   De préférence, X⁻ est un anion tel que le chlorure ou le bromure.
Ces polymères ont une masse moléculaire moyenne en nombre généralement comprise entre 1000 et 100000.
Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule (VIII) suivante: 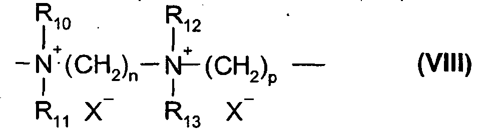 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
**(11)** Les polymères de poly(ammonium quaternaire) constitués de motifs récurrents de formule (IX): 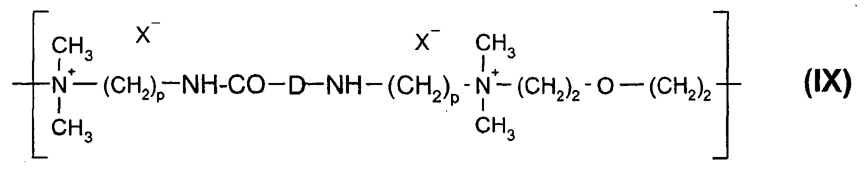 dans laquelle p désigne un nombre entier variant de 1 à 6 environ, D peut être nul ou peut représenter un groupement -(CH₂)ᵣ-CO- dans lequel r désigne un nombre égal à 4 ou à 7, X⁻ est un anion ;
   De tels polymères peuvent être préparés selon les procédés décrits dans les brevets américains 4 157 388, 4 702 906, 4 719 282. Ils sont notamment décrits dans la demande de brevet EP-A-122 324.
**(12)** Les polymères quaternaires de vinylpyrrolidone et de vinylimidazole.
**(13)** Les polyamines comme le produit référencé sous le nom de " POLYETHYLENEGLYCOL (15) TALLOW POLYAMINE " dans le dictionnaire CTFA.
**(14)** Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

### Polymères amphotères

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.
Les polymères amphotères répondant à la définition donnée ci-dessus plus particulièrement préférés sont choisis parmi les polymères suivants :
**(1)** Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium.
   Le composé vinylique peut être également un sel de dialkyidiallylammonium tel que le chlorure de diméthyldiallylammonium.
**(2)** Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butyl aminoéthyle, de N,N'-diméthylaminoéthyle, de N-tertio-butylaminoéthyle.
**(3)** Les polyaminoamides réticulés et alcoylés partiellement ou totalement dérivant de polyaminoamides de formule générale :

   ⁅CO―R₁₉―CO―Z⁆ (X)

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono
   ou bis-secondaire et de préférence représente :
   a) dans les proportions de 60 à 100 moles %, le radical 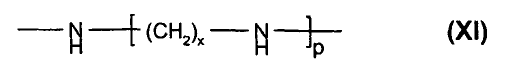 où x=2 et p=2 ou 3, ou bien x=3 et p=2 ce radical dérivant de la diéthylène triamine, de la triéthylène tétraamine ou de la dipropylène triamine;
   b) dans les proportions de 0 à 40 moles % le radical (XI) ci-dessus, dans lequel x=2 et p=1 et qui dérive de l'éthylènediamine, ou le radical dérivant de la pipérazine: 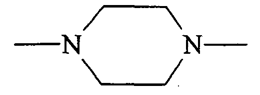
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
**(4)** Les polymères comportant des motifs zwittérioniques de formule : 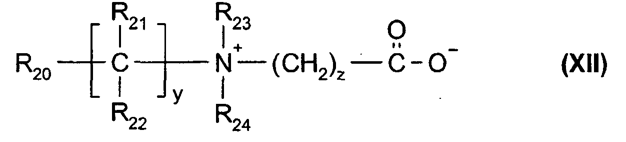 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.
**(5)** les polymères dérivés du chitosane comportant des motifs monomères répondant aux formules (XIII), (XIV), (XV) suivantes : 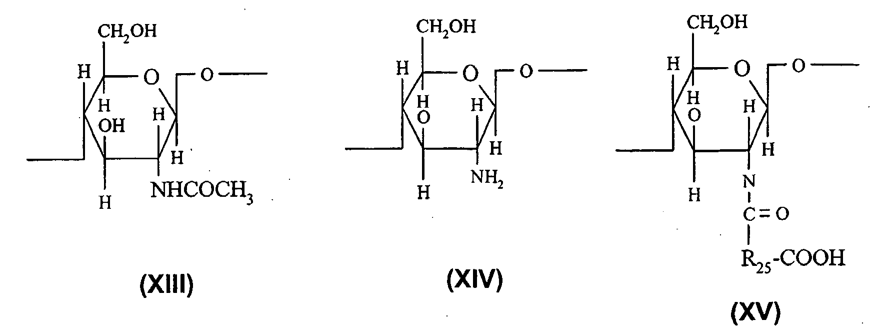 le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), R₂₅ représente un radical de formule : 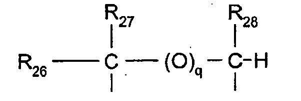 dans laquelle q désigne zéro ou 1 ; si q=0, R₂₆, R₂₇ et R₂₈, identiques ou différents, représentent chacun un atome d'hydrogène, un reste méthyle, hydroxyle, acétoxy ou amino, un reste monoalcoylamine
   ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
**(6)** Les polymères dérivés de la N-carboxyalkylation du chitosane comme le N-carboxyméthyl chitosane ou le N-carboxybutyl chitosane.
**(7)** Les polymères répondant à la formule générale (XI) tels que ceux décrits par exemple dans le brevet français 1 400 366 : 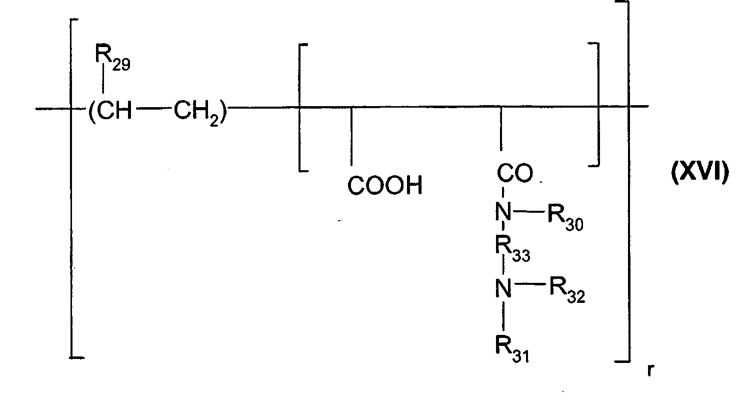 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁ désigne l'hydrogène ou un radical alkyle inférieur tel que méthyle, éthyle, R₃₂ désigne un radical alkyle inférieur tel que méthyle, éthyle ou un radical répondant à la formule :
   -R₃₃-N(R₃₁)₂, R₃₃ représentant un groupement -CH₂-CH₂-, -CH₂-CH₂-CH₂-, -CH₂-CH(CH₃)-, R₃₁ ayant les significations mentionnées ci-dessus,
   ainsi que les homologues supérieurs de ces radicaux et contenant jusqu'à 6 atomes de carbone,
   r est tel que le poids moléculaire est compris entre 500 et 6000000 et de préférence entre 1000 et 1000000.
**(8)** Des polymères amphotères du type -D-X-D-X- choisis parmi :
   a) les polymères obtenus par action de l'acide chloracétique ou le chloracétate de sodium sur les composés comportant au moins un motif de formule :

      -D-X-D-X-D- (XVII)

      où D désigne un radical 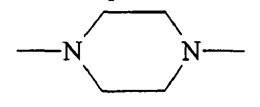 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- (XVIII)
   où D désigne un radical 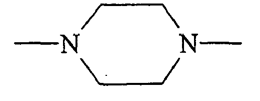 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique
   ou du chloracétate de soude.
**(9)** Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Parmi tous les polymères cationiques ou amphotères utilisables selon la présente invention, on préfère notamment :
(i)parmi les cationiques :
   - l'homopolymère de chlorure de diméthyldiallylammonium vendu sous la dénomination MERQUAT 100DRY par la société MERCK;
   - les copolymères de chlorure de diméthyldiallylammonium et d'acrylamide vendus sous la dénomination MERQUAT 2200 par la société CALGON;
   - les polymères de type poly(ammonium quaternaire) préparés et décrits dans le brevet français 2 270 846, constitués de motifs récurrents de formules (W) et (U) suivantes : 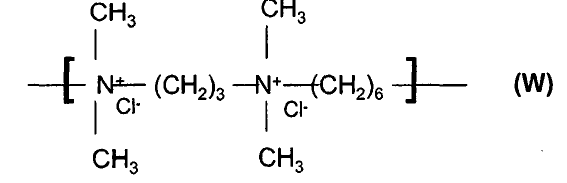 et notamment ceux dont le poids moléculaire, déterminé par chromatographie par perméation de gel, est compris entre 9500 et 9900; 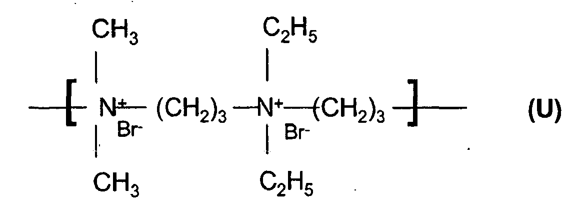 et notamment ceux dont le poids moléculaire, déterminé par chromatographie par perméation de gel, est d'environ 1200;
   - les polymères de type poly(ammonium quaternaire) de la famille (11) et de formule (IX) dans laquelle X⁻ désigne le Chlore, et notamment ceux dont la masse moléculaire moyenne en poids est inférieure à 100 000, de préférence inférieure ou égale à 50 000;
(ii)parmi les polymères amphotères:
   - le copolymère chlorure de diméthyldiallylammonium/acide acrylique (80/20) commercialisé sous la dénomination MERQUAT 280 DRY par la société CALGON (dénomination CTFA : POLYQUATERNIUM 22);
   - le copolymère chlorure de diméthyldiallylammonium/acide acrylique (95/5) commercialisé sous la dénomination MERQUAT 295 DRY par la société CALGON (dénomination CTFA : POLYQUATERNIUM 22);
   - le copolymère de chlorure de méthacrylamidopropyltrimonium, d'acide acrylique et d'acrylate d'éthyle, commercialisé sous la dénomination MERQUAT 2001 par la société CALGON (dénomination CTFA ; POLYQUATERNIUM 47); et
   - le terpolymère acrylamide/chlorure de diméthyldiallylammonium/acide acrylique, commercialisé sous la dénomination MERQUAT PLUS 3330 DRY par la société CALGON (dénomination CTFA : POLYQUATERNIUM 39).

Lorsqu'ils ils sont présents dans les pâtes décolorantes de la présente invention, les polymères cationiques et/ou amphotères sont présents dans une proportion pondérale inférieure ou égale à 20% par rapport au poids total de ladite composition et de préférence inférieure ou égale à 8%.

### Préparation de la pâte décolorante selon l'invention

Selon la présente invention, on prépare la pâte décolorante en dispersant, sous action mécanique, l'ensemble des composés pulvérulents dans le polydécène selon l'invention,
dans lequel on a préalablement dispersé ou mélangé les autres composés liquides de la composition.
On peut également préparer la pâte par extrusion, en introduisant les phases liquides et solides de la composition dans l'extrudeur, puis en les mélangeant à une température inférieure à 25°C à l'aide d'un système bi-vis co-rotatives composé d'éléments de transport et de malaxage.

La pâte décolorante ainsi préparée est utilisée pour la préparation d'une composition prête à l'emploi qui résulte du mélange extemporané de ladite pâte avec une composition aqueuse de peroxyde d'hydrogène.
La pâte est ainsi mélangée avec environ 0,5 à 10 équivalents en poids d'une composition aqueuse de peroxyde d'hydrogène qui peut être une solution, une émulsion ou un gel, ayant une concentration pondérale allant de 2 à 12% en peroxyde d'hydrogène.
Ce mélange doit se faire immédiatement avant l'application du produit sur les cheveux.

La composition aqueuse de peroxyde d'hydrogène présente de préférence un pH inférieur à 7.

Le pH acide garantit la stabilité du peroxyde d'hydrogène dans la composition.
Il peut être obtenu à l'aide d'agents acidifiants tels que par exemple l'acide chlorhydrique, l'acide acétique, l'acide éthydronique, l'acide phosphorique, l'acide lactique ou l'acide borique, et il peut être ajusté classiquement par ajout soit d'agents basifiants, tels que par exemple, l'ammoniaque, la monoéthanolamine, la diéthanolamine, la triéthanolamine, l'isopropanolamine, le 1,3-diamino-propane, un (bi)carbonate alcalin ou d'ammonium, un carbonate organique tel que le carbonate de guanidine, ou bien encore un hydroxyde alcalin, tous ces composés pouvant bien entendu être pris seuls ou en mélange.

La composition aqueuse de peroxyde d'hydrogène peut encore contenir des agents conservateurs, des colorants, des parfums, des agents antimousse, des agents stabilisants du peroxyde d'hydrogène tels que notamment le pyrophosphate de sodium, le stannate de sodium et le salicylate de sodium ainsi que des agents séquestrants tels que par exemple l'acide éthylènediamine tétraacétique (EDTA) ou le Pentasodium Pentetate (dénomination CTFA).

Bien entendu, l'homme de l'art veillera à choisir le ou les éventuels composés complémentaires mentionnés ci-avant, de manière telle que les propriétés avantageuses attachées intrinsèquement à la pâte anhydre de décoloration ou à la composition de décoloration prête à l'emploi selon l'invention ne soient pas, ou substantiellement pas, altérées par la ou les adjonctions envisagées.

Le pH de la composition de décoloration prête à l'emploi est généralement compris entre les valeurs 4 et 12. Il est de préférence compris entre 7 et 11,5 et encore plus préférentiellement entre 8 et 11.

De préférence, le procédé de décoloration selon l'invention consiste à mélanger immédiatement avant emploi une pâte anhydre telle que décrite selon la présente invention avec une composition aqueuse de peroxyde d'hydrogène telle que décrite selon la présente invention, à appliquer la composition de décoloration prête à l'emploi ainsi obtenue sur la zone des fibres kératiniques humaines (sèches ou humides) à décolorer, et à la laisser agir pendant un temps de pause suffisant pour obtenir la décoloration recherchée, variant de préférence, de 1 à 60 minutes environ, et plus préférentiellement de 10 à 45 minutes environ, à éliminer le mélange décolorant par rinçage à l'eau, suivi d'un lavage avec un shampooing, puis éventuellement d'un séchage.

Des exemples concrets illustrant l'invention sont indiqués ci-après, sans pour autant présenter un caractère limitatif.

### EXEMPLES DE PATES ANHYDRES DE DECOLORATION :

On a préparé les 6 pâtes anhydres 1 à 6 suivantes pour la décoloration des cheveux (quantités exprimées en grammes de Matière Active) :

| **Exemples** | **Selon l'invention** | | | | | **Art antérieur** |
|---|---|---|---|---|---|---|
| | **1** | **2** | **3** | **4** | **5** | **6** |
| Persulfate de Potassium | 36,1 | 35,9 | 36,9 | 37,1 | 36,1 | 38,3 |
| Persulfate de Sodium | 6 | 6 | 6 | 6 | 6 | 6 |
| Disilicate de Sodium | 15 | 15 | 15 | 15 | 15 | 15 |
| Chlorure d'ammonium | 4,2 | 4,2 | 4,2 | 4,2 | 4,2 | 2,2 |
| Métasilicate de sodium | 3 | 3 | 3 | 3 | 3 | 3 |
| Sulfate d'ammonium | | 2 | | | | 2 |
| EDTA (séquestrant) | 0,2 | 0,2 | 0,2 | 0,2 | 0,2 | 0,2 |
| Polymère amphiphile non-ionique à chaîne grasse: | | | | | | |
| Ser-ad FX 1100 vendu par la Société Servo Delden | 0,5 | 0,5 | 2 | | | |
| Polymère amphiphile anionique à chaîne grasse: | | | | | | |
| copolymère réticulé d'acide acrylique et de méthacrylate d'alkyle en C₁₀₋₃₀, (Carbopol ETD 2020 vendu par la Société Goodrich) | | | | 0,5 | 0,5 | 0,5 |
| Polymère épaississant hydrosoluble: | | | | | | |
| Primogel vendu par Avebe | 1 | 1 | 2 | 1 | 1 | 1 |
| Polymère épaississant hydrosoluble: | | | | | | |
| Gomme de guar non ionique non modifiée (Guargel D/15 vendu par la société Goodrich) | 0,5 | 0,5 | | | 0,5 | 3 |
| Gomme de xanthane (Keltrol BT vendu par la société Nutrasweet Kelco | 3 | 3 | | 2 | 3 | |
| Tensio-actif anionique: | | | | | | |
| Cétostéaryl sulfate de Sodium | | | 1 | | | 2,5 |
| Tensio-actif anionique: | | | | | | |
| Lauryl sulfate de sodium | 4 | 4 | 3 | 4 | 4 | |
| Stéarate de sodium | | | 2 | | | 1 |
| Stéarate de magnésium. | 2 | 2 | | 2 | 2 | |
| Polyvinylpyrrolidone | | | | | 0,5 | 0,5 |
| Oxyde de titane | 1 | 1 | 1 | 1 | 0,5 | 1 |
| Cire d'abeille | | | | | | 1 |
| Colorant (ultramarine) | | 0,2 | 0,2 | 0,5 | | |
| Palmitate d'isopropyle vendu sous la dénomination Isopropyl Palmitate par la société Cognis | | | | | | 21,8 |
| Polydécène vendu sous la dénomination Silkflo 366 NF Polydecene par la société Amoco Chemical | | 18 | | | | |
| Polydécène vendu sous la dénomination | | | | | | |
| Nexbase 2006 FG par la société Fortum | 18 | | 22,5 | 22,5 | 18 | |
| Silice amorphe | | | 0,5 | | | 1 |
| Silice pyrogénée à caractère hydrophobe, (Aerosil® R972 vendu par la société Degussa Hûls) | | 0,5 | 0,7 | | | |
| Silice pyrogénée à caractère hydrophile, (Aerosil® 300 vendu par la société Degussa Hûls) | 0,5 | 0,5 | | 0,3 | 0,5 | |
| Polymère bloc selon l'invention : vendu sous la dénomination Geahlene 200 par la société Penreco | 5 | 5 | | | 5 | |

Les pâtes des exemples 1 à 5 selon l'invention se sont révélées moins sensibles à la succession des chocs thermiques ainsi qu'à l'exposition aux températures inférieures à 4°C et supérieures à 45°C, que la pâte de l'exemple 6 de l'art antérieur à base de cire et d'ester isopropylique d'acide palmitique.
En outre, les pâtes des exemples 1 à 5, mélangées avec une composition aqueuse de peroxyde d'hydrogène à 9%, ont conduit à des décolorations plus puissantes et plus homogènes en ne laissant les cheveux ni gras ni rêches.

## Revendications

1. Pâte anhydre pour la décoloration des fibres kératiniques humaines, et plus particulièrement des cheveux, comprenant au moins un sel peroxygéné, au moins un agent alcalin, **caractérisée par le fait qu'**elle comprend 15 à 35% en poids d'au moins un polydécène de formule C₁₀ₙ H_{[(20n)+2]} dans laquelle n varie de 3 à 9 et de préférence de 3 à 7, et 0,01 à 10% en poids d'au moins un gélifiant choisi parmi les silices pyrogénées à caractère hydrophile ou hydrophobe ou un copolymère di-, tri-, multi- ou radial bloc composé de segments de type styrène monomère et de segments de type monomère ou comonomère thermoplastique.

2. Pâte selon la revendication 1, **caractérisée par le fait que** le ou les polydécènes sont présents à une concentration allant de 15 à 30% en poids par rapport au poids total de la pâte.

3. Pâte selon l'une quelconque des revendications précédentes, **caractérisée par le fait que** le ou les agents gélifiants sont présents à une concentration allant de 0,01 à 5% en poids par rapport au poids total de la pâte.

4. Pâte selon la revendication 3, **caractérisée par le fait que** le ou les agents gélifiants sont présents à une concentration allant de 0,1 à 2,5% en poids par rapport au poids total de la pâte.

5. Pâte selon l'une quelconque des revendications précédentes, **caractérisée par le fait que** dans le copolymère bloc défini selon la revendication 1, les segments de type monomère ou comonomère thermoplastique, sont des segments éthylène/alkylène en C₃-C₄.

6. Pâte selon la revendication 5, **caractérisée par le fait que** le copolymère bloc est un copolymère bloc hydrogéné à blocs styrène et à blocs éthylène/alkylène en C₃-C₄.

7. Pâte selon l'une quelconque des revendications précédentes, **caractérisée par le fait que** les sels peroxygénés sont choisis parmi les persulfates de sodium ou de potassium.

8. Pâte selon l'une quelconque des revendications précédentes, **caractérisée par le fait que** les sels peroxygénés sont présents à une concentration allant de 10 à 70% en poids par rapport au poids total de la pâte.

9. Pâte selon l'une quelconque des revendications précédentes, **caractérisée par le fait que** les agents alcalins sont présents à une concentration allant de 0,01 à 40% en poids par rapport au poids total de la pâte.

10. Pâte selon l'une quelconque des revendications précédentes, **caractérisée par le fait qu'**elle comprend 0,01 à 30% en poids d'au moins un polymère amphiphile non ionique ou anionique comportant au moins une chaîne grasse par rapport au poids total de la pâte.

11. Pâte anhydre pour la décoloration des fibres kératiniques humaines, et plus particulièrement des cheveux, comprenant 10 à 70% en poids d'au moins un persulfate de sodium ou de potassium, 0,01 à 40% en poids d'au moins un agent alcalin, 15 à 35% en poids d'au moins un polydécène de formule C₁₀ₙ H_{[(20n)+2]} dans laquelle n varie de 3 à 9 et de préférence de 3 à 7, et 0,01 à 10% en poids d'au moins un gélifiant choisi parmi les silices pyrogénées à caractère hydrophile ou hydrophobe et les copolymères blocs hydrogénés à blocs styrène et à blocs éthylène/alkylène en C₃-C₄.

12. Utilisation de la pâte définie à l'une quelconque des revendications 1 à 11, dans une ou pour la préparation d'une composition de décoloration prête à l'emploi par addition extemporanée d'une composition aqueuse de peroxyde d'hydrogène.

13. Procédé de décoloration des fibres kératiniques humaines et en particulièrement des cheveux, comprenant les étapes consistant :
- à mélanger, immédiatement avant emploi, une pâte anhydre définie selon l'une quelconque des revendications 1 à 11, avec une composition aqueuse de peroxyde d'hydrogène,
- à appliquer le mélange obtenu sur la zone des fibres à décolorer,
- à laisser reposer pendant un temps suffisant pour obtenir la décoloration recherchée,
- à éliminer le mélange décolorant par rinçage à l'eau suivi d'un lavage avec un shampooing, puis éventuellement d'un séchage.

14. Dispositif à plusieurs compartiments, ou "kit", pour la décoloration des fibres kératiniques humaines et plus particulièrement des cheveux, **caractérisé par le fait qu'**il comporte au moins deux compartiments dont l'un comprend une pâte anhydre selon l'une quelconque des revendications 1 à 11, et l'autre une composition aqueuse de peroxyde d'hydrogène.

## Claims

1. Anhydrous paste for bleaching human keratin fibres, and more particularly the hair, comprising at least one peroxygenated salt and at least one alkaline agent, **characterized in that** it comprises 15% to 35% by weight of at least one polydecene of formula C₁₀ₙH_{[(20n)+2]} in which n ranges from 13 to 9 and preferably from 3 to 7, and 0.01% to 10% by weight of at least one gelling agent chosen from fumed silicas of hydrophilic or hydrophobic nature or a diblock, triblock, multiblock or radial-block copolymer composed of segments of styrene monomer type and segments of thermoplastic monomer or comononer type.

2. Paste according to Claim 1, **characterised in that** the polydecene(s) is (are) present in a concentration ranging from 15% to 30% by weight relative to the total weight of the paste.

3. Paste according to either of the preceding claims, **characterized in that** the gelling agent (s) is (are) present in a concentration ranging from 0.01% to 5% by weight relative to the total weight of the paste.

4. Paste according to claim 3, **characterised in that** the gelling agent(s) is (are) present in a concentration ranging from 0.1% to 2.5% by weight relative to the total weight of the paste.

5. Paste according to any one of the preceding claims, **characterized in that**, in the block copolymer defined according to Claim 1, the segments of thermoplastic monomer or comonomer type are C₃-C₄ ethylene/alkylene segments.

6. Paste according two Claim 5, **characterized in that** the block copolymer is a hydrogenated block copolymer containing styrene blocks and C₃-C₄ ethylene/alkylene blocks.

7. Paste according to any one of the preceding claims, **characterized in that** the peroxygenated salts are chosen from sodium persulphate and potassium persulphate.

8. Paste according to and one of the preceding claims, **characterised in that** the peroxygenated salts are present in a concentration ranging from 10% to 70% by weight relative to the total weight of the paste.

9. Paste according to any one of the preceding claims, **characterised in that** the alkaline agents are present in a concentration ranging from 0.01% to 40% by weight relative to the total weight of the paste.

10. Paste according to any one of the preceding claims, **characterised in that** it comprises 0.01% to 30% by weight of at least one nonionic or anionic amphiphilic polymer comprising at least one fatty chain relative to the total weight of the paste,

11. Anhydrous paste for bleaching human keratin fibres, and more particularly the hair, comprising 10% to 70% by weight of at least one sodium persulphate or potassium persulphate, 0.01% to 40% by weight of at least one alkaline agent, 15% to 35% by weight of at leat one polydecene of formula C₁₀ₙH_{[(20n)+2]} in which n ranges from 3 to 9 and preferably from 3 to 7, and 0.01% to 10% by weight of at least one gelling agent chosen from fumed silicas of hydrophilic or hydrophobic nature and hydrogenated block copolymers containing styrene blocks and C₃-C₄ ethylene/alkylene blocks.

12. Use of the paste defined in any one of Claims 1 to 11, in or for the preparation of a ready-to-use bleaching composition by extemporaneous addition of an aqueous hydrogen peroxide composition.

13. Process for bleaching human keratin fibres, and in particular the hair, comprising the steps consisting in:
- mixing, immediately before use, an anhydrous paste defined according to any one of Claims 1 to 11 with an aqueous hydrogen peroxide composition,
- applying the mixture obtained to the area of fibres to be bleached,
- leaving the mixture to act for a period of time that is sufficient to obtain the desired bleaching,
- removing the bleaching mixture by rinsing with water, followed by washing with a shampoo and then optionally drying.

14. Multi-compartment devise, or "kit", for bleaching human keratin fibres and more particularly the hair, **characterised in that** it comprises at least two compartments, one of which comprises an anhydrous paste according to any one of Claims 1 to 11, and the other of which comprises an aqueous hydrogen peroxide composition.

## Patentansprüche

1. Wasserfreie Paste zum Entfärben von menschlichen Keratinfasern und insbesondere von Haaren, umfassend mindestens ein Peroxysalz und mindestens ein alkalisches Mittel, **dadurch gekennzeichnet, daß** sie 15 bis 35 Gew.-% mindestens eines Polydecens der Formel C₁₀ₙH_{[(20n)+2]}, worin n im Bereich von 3 bis 9 und vorzugsweise 3 bis 7 liegt, und 0,01 bis 10 Gew.-% mindestens eines Gelbildners, der unter pyrogenen Kieselsäuren mit hydrophilem oder hydrophobem Charakter oder einem Diblöck-, Triblock-, Multiblock*-* oder Radialblockcopolymer aus Segmenten vom Typ Styrolmonomer und Segmenten vom Typ thermoplastisches Monomer oder Comonomer ausgewählt ist, umfaßt.

2. Paste nach Anspruch 1, **dadurch gekennzeichnet, daß** das oder die Polydecene in einer Konzentration von 15 bis 30 Gew.-%, bezogen auf das Gesamtgewicht der Paste, vorliegen.

3. Paste nach einem der vorhergehenden Anspruche, **dadurch gekennzeichnet, daß** der oder die Gelbildner in einer Konzentration von 0,01 bis 5 Gew.-%, bezogen auf das Gesamtgewicht der Paste, vorliegen.

4. Paste nach Anspruch 3, **dadurch gekennzeichnet, daß** der oder die Gelbildner in eines Konzentration von 0,1 bis 2,5 Gew,-%, bezogen auf das Gesamtgewicht der Paste, vorliegen.

5. Paste nach einem der vorhergehenden Ansprüche, **dadurch gekennzeichnet, daß** es sich in dem gemäß Anspruch 1 definierten Blockcopolymer bei den Segmenten vom Typ thermoplastisches Monomer oder Comonomer um Ethylen/C₃-C₄-Alkylen-Segments handelt.

6. Paste nach Anspruch 5, **dadurch gekennzeichnet, daß** es sich bei dem Blockcopolymer um ein hydriertes Blockcopolymer mit Styrolblöcken und Ethylen/C₃-C₄-Alkylen-Blöcken handelt.

7. Paste nach einem der vorhergehenden Ansprüche, **dadurch gekennzeichnet, daß** die Peroxysalze unter Natrium- und Kaliumpersulfat ausgewählt sind.

8. Paste nach einem der vorhergehenden Ansprüche, **dadurch gekennzeichnet, daß** die Peroxysalze in einer Konzentration von 10 bis 70 Gew, -%, bezogen auf das Gesamtgewicht der Paste, vorliegen.

9. Paste nach einem der vorhergehenden Anspruche, **dadurch gekennzeichnet, daß** die alkalischen Mittel in einer Konzentration von 0,01 bis 40 Gew.-%, besogen auf das Gesamtgewicht der Paste, vorliegen.

10. Paste nach einem der vorhergehenden Anspruche, **dadurch gekennzeichnet, daß** sie 0,01 bis 30 Gew.-% mindestens eines nichtionischen oder anionischen amphiphilen Polymers mit mindestens einer Fettkette, bezogen auf das Gesamtgewicht der Paste, umfaßt.

11. Wasserfreie Paste zum Entfärben von menschlichen Keratinfasern und insbesondere von Haaren, umfassend 10 bis 70 Gew.-% Natrium- und/oder Kaliumpersulfat, 0,01 bis 40 Gew.-% mindestens eines alkalischen Mittels, 15 bis 35 Gew.-% mindestens eines Polydecens der Formel C₁₀ₙH_{[(20n)+2]}, worin n im Bereich von 3 bis 9 und vorzugsweise 3 bis 7 liegt, und 0,01 bis 10 Gew.-% mindestens eines Gelbildners, der unter pyrogenen Kieselsäuren mit hydrophilem oder hydrophobem Charakter und hydrierten Blockcopolymeren mit Styrolblöcken und Ethylen/C₃-C₄-Alkylen-Blocken ausgewählt ist.

12. Verwendung der Paste gemäß einem der Ansprüche 1 bis 11 in oder zum Herstellen einer gebrauchsfertigen Entfärbezusammensetzung durch unmittelbar vor dem Gebrauch erfolgende: Zugabe einer wäßrigen Wasserstoffperoxidzusammensetzung.

13. Verfahren zum Entfärben von menschlichen Keratinfasern und insbesondere von Haaren, bei dem man:
- unmittelbar vor dem Gebrauch eine wasserfreie Paste gemäß einem der Anspruche 1 bis 11 mit einer wäßrigen Waserstoffperoxidzusammensetzung mischt,
- die erhaltene Mischung auf den zu entfärbenden Bereich der Fasern aufbringt,
- die Mischung über einen zum Erhalt der gewünschten Entfärbung ausreichenden Zeitraum einwirken läßt,
- die Entfärbungsmischung mit Wasser abspült, dann mit einem Shampoo wäscht und danach gegebenenfalls trocknet.

14. Vorrichtung mit mehreren Kompartimenten oder "Kit" zum Entfärben von menschlichen Keratinfasern und insbesondere von Haaren, **dadurch gekennzeichnet, daß** sie mindestens zwei Kompartimente aufweist, von denen eines eine wasserfreie Paste gemäß einem der Ansprüche 1 bis 11 und das andere eine wäßrige Wasserstoffperoxidzusammensetzung enthält.
